# EUROPEAN PATENT APPLICATION

(11) **EP 4 837 579 A2**
(43) Date of publication of application: **11.11.2026**
(21) Application number: 26176955.8
(22) Date of filing: 06.05.2026
(51) Int. Cl.: A61K 31/5575, A61K 9/00, A61K 9/08, A61K 47/06, A61K 47/14, A61P 27/06

(54) **OPHTHALMIC PHARMACEUTICAL COMPOSITION COMPRISING LATANOPROSTENE BUNOD AND USE THEREOF**

(30) Priority: 08.05.2025 TW 114117389; 06.08.2025 TW 114129978
(71) Applicant: Caravel Oculus Inc., Taipei City 105404 (TW)
(72) Inventor: LEE, CHIEN-HUNG, 105404 Taipei City (TW); CHUANG, HSUN-YUEH, 105404 Taipei City (TW); PENG, SHIH-FENG, 105404 Taipei City (TW); LIN, CHENG-CHIEH, 105404 Taipei City (TW)
(74) Representative: Patentwerk B.V.

(57) **Abstract**

The present disclosure provides an ophthalmic pharmaceutical composition and the use thereof, where the ophthalmic pharmaceutical composition includes: 0.006% w/v to 0.121 % w/v of latanoprostene bunod (LBN) or a pharmaceutically acceptable salt thereof; at least one excipient, including an oil phase and a liquid vehicle of semifluorinated alkanes; where the composition is microbiologically stable, substantially water-free and preservative-free.

## Description

### BACKGROUND OF THE INVENTION

### 1. Field of the Invention

The present disclosure belongs to the field of pharmaceuticals, and in particular relates to an ophthalmic pharmaceutical composition and use thereof, specifically use of the ophthalmic pharmaceutical composition for prevention, treatment, or alleviation of eye diseases in an individual.

### 2. Description of the Related Art

Glaucoma is optic nerve damage caused by high intraocular pressure. Besides glaucoma, high intraocular pressure may also lead to other related diseases, such as dry eye symptoms, eye redness or non-arterial anterior ischemic optic neuropathy (NAION). The intraocular pressure is mainly a balance between the secretion rate and the outflow rate of aqueous humor in eyeballs, and is also affected by the thickness and hardness of corneae. The normal intraocular pressure is between 10 mmHg and 22 mmHg, and generally, the intraocular pressure is about 15 mmHg to 16 mmHg, and the fluctuations can reach 6 mmHg. In addition, the intraocular pressure will also change with physiological factors, such as exercise, heartbeat, respiration, fluid intake or certain systemic or topical medications.

It has been previously found that several prostaglandin analogs can effectively reduce intraocular pressure as topical medications, such as latanoprost, bimatoprost, tafluprost, travoprost, and unoprostone. Latanoprost, travoprost, and tafluprost are all a potent and highly selective PGF2α receptor agonist. Their effects involves significantly increasing the amount of aqueous humor outflowing from a uveoscleral pathway to reduce the intraocular pressure, and also potentially increasing the amount of aqueous humor outflowing from a trabecular meshwork pathway to some extent.

Nowadays, various ophthalmic drug formulas containing prostaglandin analogs have been developed and marketed. Most common ophthalmic drug formulas or eye drop solutions contain preservatives. There are many shortcomings in the formula of an eye drop solution containing preservatives, especially during long-term use for glaucoma treatment and the like, where irritation or allergic reaction can be caused. The most common preservative in the above-mentioned formula is benzalkonium chloride, which is a quaternary ammonium compound and is often related to irritative and toxic reactions. A unit-dose package without preservatives can avoid this problem, but the cost is high. Not only does each dose require a separate container, but also additional medical solution filling is required, resulting in waste of a medical solution due to significant residual medical solution in the container. Even if the medical solution is successfully instilled into the eyes, only a portion of the medical solution will take effect because of the limited capacity of the lacrimal sac, with portions expelled via blinking or absorbed through the nasolacrimal duct, potentially causing adverse drug reactions.

For poorly water-soluble active ingredients in pharmaceutical formulas, oily eye drop preparations are generally used. Such preparations can dissolve poorly water-soluble drugs more effectively and usually does not require the addition of a pH regulator or osmotic pressure regulator. For example, poorly water-soluble drugs such as cyclosporine, even when used as oil in water emulsions where the active ingredients exhibit some solubility in an oil phase, face the problem of limited drug load due to the fact that the oil phase is merely continuously dispersed in a water phase. Meanwhile, oily preparations will have a negative impact on vision. Whether they are used as pure oily solvents or oil in water emulsions, their refractive indexes are significantly different from those of physiological tears, leading to visual interference and blurred vision. In addition, the oil in water emulsions are more complex and difficult to prepare than single-phase systems (such as aqueous solvents or oily solvents), especially in sterile manufacturing, the emulsions generally cannot withstand high-temperature sterilization treatment, as this may adversely affect their physical properties. For another, the sterile operation process is also quite complex, costly, and has a relatively high risk of contamination.

### BRIEF SUMMARY OF THE INVENTION

Based on the above, it can be understood that there are still many problems in the existing ophthalmic preparations that are required to be solved urgently. In view of this, the inventors proposed a solution, which is to provide a water-free and preservative-free ophthalmic pharmaceutical composition and use thereof, where in addition to being highly stable, the ophthalmic pharmaceutical composition can improve the conventional side effects and achieve better efficacy at a lower concentration.

According to an embodiment of the present disclosure, the composition includes 0.006% w/v to 0.040% w/v of latanoprostene bunod (LBN) or a pharmaceutically acceptable salt thereof; at least one excipient, including an oil phase and a liquid vehicle of semifluorinated alkanes (SFAs), where the composition is microbiologically stable, and is water-free and preservative-free; the composition is formulated into a solution, an emulsion, or a suspension; where, in the excipient, the concentration of the oil phase is 10% w/v to 50% w/v; and the solubility of the latanoprostene bunod in the excipient is 0.56 mg/mL to 1.21 mg/mL.

According to an embodiment of the present disclosure, in the excipient, the concentration of the oil phase is 15% w/v, and the solubility of the latanoprostene bunod in the excipient is 0.56 mg/mL.

According to an embodiment of the present disclosure, the oil phase is selected from triglyceride oil, mineral oil, medium-chain triglyceride (MCT), oily fatty acid, isopropyl myristate, oily fatty alcohol, sorbitan ester, oily sucrose ester, or other oily substances that are physiologically acceptable to the eyes.

According to an embodiment of the present disclosure, in the excipient, the concentration of the oil phase is 15% w/v to 30% w/v.

According to an embodiment of the present disclosure, the refractive index of the semifluorinated alkanes ranges from 1.29 to 1.35 at 20°C.

According to an embodiment of the present disclosure, the semifluorinated alkanes are linear or branched alkanes in which a non-fluorinated hydrocarbon segment is linked to a perfluorinated hydrocarbon segment.

According to an embodiment of the present disclosure, the general formula of the semifluorinated alkanes is RFRH, where RF represents the perfluorinated hydrocarbon segment, RH represents the non-fluorinated hydrocarbon segment, and RF and RH are each of a linear structure, and each have 3 to 20 carbon atoms.

According to an embodiment of the present disclosure, the perfluorinated hydrocarbon segment includes 4 to 12 carbon atoms, and the non-fluorinated hydrocarbon segment includes 4 to 10 carbon atoms.

According to an embodiment of the present disclosure, the semifluorinated alkanes are selected from the group consisting of F4H4, F4H5, F4H6, F4H7, F4H8, F6H2, F6H4, F6H6, F6H7, F6H8 (perfluorohexyloctane), F6H9, and F6H10.

According to an embodiment of the present disclosure, the excipient further includes: a cosolvent and/or a solvent.

According to an embodiment of the present disclosure, the cosolvent is an organic solvent selected from glycerol, propylene glycol, polyethylene glycol, and ethanol.

In another aspect, the present disclosure provides use of the above-mentioned ophthalmic pharmaceutical composition for prevention, treatment, or alleviation of eye diseases in an individual, where the eye diseases include elevated intraocular pressure-related symptoms, glaucoma, dry eye symptoms, eye redness or non-arterial anterior ischemic optic neuropathy (NAION).

According to an embodiment of the present disclosure, the prevention, treatment, or alleviation of glaucoma is lowering the intraocular pressure in a patient.

According to an embodiment of the present disclosure, the dry eye symptoms include: eye dryness, photophobia, eye redness with bloodshot appearance, eye soreness, ocular foreign body sensation, stringy eye discharge, blurred vision, and/or poor vision.

According to an embodiment of the present disclosure, the prevention, treatment, or alleviation of non-arterial anterior ischemic optic neuropathy (NAION) includes: administering the ophthalmic pharmaceutical composition to an individual by contact with the eyes or injection into the eyes, where when the ophthalmic pharmaceutical composition is administered to the individual by contact with the eyes, the concentration of latanoprostene bunod in the ophthalmic pharmaceutical composition is 0.006% w/v to 0.04% w/v, and the ophthalmic pharmaceutical composition is administered three times daily for three weeks; and when the ophthalmic pharmaceutical composition is administered to the individual by injection into the eyes, the concentration of latanoprostene bunod in the ophthalmic pharmaceutical composition is 0.04% w/v, and a single injection was employed.

To sum up, the ophthalmic pharmaceutical composition provided by the present disclosure is water-free and preservative-free. Furthermore, the ophthalmic pharmaceutical composition is highly stable and can improve the conventional side effects, and can achieve better efficacy at a lower concentration.

### BRIEF DESCRIPTION OF THE DRAWINGS

FIG. 1 is a comparative graph of intraocular pressure lowering effects between an ophthalmic pharmaceutical composition (CA202L) and a commercially available drug in an embodiment of the present disclosure.
FIG. 2 is a comparative graph of intraocular pressure changes between an ophthalmic pharmaceutical composition (CA202L) and a commercially available drug in an embodiment of the present disclosure.
FIG. 3 is a self-rating dry eye symptom scale (OSDI).
FIG. 4 shows comparison data of red eye severity between an ophthalmic pharmaceutical composition (CA202L) and a commercially available drug according to an embodiment of the present disclosure.

### DETAILED DESCRIPTION OF THE INVENTION

The present disclosure provides an ophthalmic pharmaceutical composition. The composition includes: a therapeutically effective amount of prostaglandin analog; and at least one excipient, including an oil phase and a liquid vehicle of semifluorinated alkanes. The ophthalmic pharmaceutical composition is microbiologically stable, and is substantially water-free and preservative-free. In addition, according to at least one embodiment of the present disclosure, the ophthalmic pharmaceutical composition is formulated as a solution, an emulsion, or a suspension.

The concentration unit "% w/v" used herein refers to the amount of solute in grams (g) contained in 100 milliliters (mL) of a solution. For example, the ophthalmic pharmaceutical composition of the present disclosure may include 0.040% w/v of latanoprostene bunod (LBN), which indicates that 0.040 g of latanoprostene bunod is contained in 100 mL of the ophthalmic pharmaceutical composition. In addition, in the excipient included in the ophthalmic pharmaceutical composition of the present disclosure, the concentration of the oil phase may be 30% w/v, which indicates that 30 g of the oil phase is contained in 100 mL of the excipient.

Specifically, to prepare an ophthalmic pharmaceutical composition including 0.040% w/v of latanoprostene bunod (LBN), 0.040 g of latanoprostene bunod may be weighed, and the excipient may be added in portions until a total volume of 100 mL is reached. In addition, to prepare an excipient in which the concentration of the oil phase is 30% w/v, 30 g of the oil phase (for example, medium-chain triglycerides (MCTs)) may be weighed, and semifluorinated alkanes (for example, F6H8) may be added in portions until a total volume of 100 mL is reached.

Specifically, the prostaglandin analog is an active ingredient, which is water-insoluble. By way of example, the prostaglandin analog includes latanoprost, bimatoprost, tafluprost, travoprost or unoprostone, preferably latanoprostene bunod (LBN) or a pharmaceutically acceptable salt thereof.

More specifically, the concentration of the prostaglandin analog is 0.005% w/v to 0.100% w/v, for example: 0.005% w/v, 0.006% w/v, 0.010% w/v, 0.012% w/v, 0.015% w/v, 0.020% w/v, 0.024% w/v, 0.025% w/v, 0.030% w/v, 0.035% w/v, 0.040% w/v, 0.045% w/v, 0.050% w/v, 0.055% w/v, 0.060% w/v, 0.065% w/v, 0.070% w/v, 0.075% w/v, 0.080% w/v, 0.085% w/v, 0.090% w/v, 0.095% w/v or 0.100% w/v, preferably 0.006% w/v to 0.040% w/v, and more preferably 0.012% w/v to 0.024% w/v.

Specifically, in the excipient, the concentration of the oil phase is 10% w/v to 50% w/v, for example, 10% w/v, 15% w/v, 20% w/v, 25% w/v, 30% w/v, 35% w/v, 40% w/v, 45% w/v, or 50% w/v, preferably 15% w/v to 30% w/v, more preferably 15% w/v to 20% w/v, and most preferably 15% w/v.

Specifically, the solubility of the prostaglandin analog in the excipient is 0.19 mg/mL to 1.21 mg/mL, for example, 0.19 mg/mL, 0.25 mg/mL, 0.30 mg/mL, 0.35 mg/mL, 0.40 mg/mL, 0.45 mg/mL, 0.50 mg/mL, 0.56 mg/mL, 0.60 mg/mL, 0.65 mg/mL, 0.70 mg/mL, 0.75 mg/mL, 0.80 mg/mL, 0.85 mg/mL, 0.90 mg/mL, 0.95 mg/mL, 1.00 mg/mL, 1.05 mg/mL, 1.10 mg/mL, 1.15 mg/mL, 1.20 mg/mL or 1.21 mg/mL, preferably 0.56 mg/mL to 1.21 mg/mL, and more preferably 0.56 mg/mL.

Specifically, the semifluorinated alkanes described herein are linear or branched alkanes in which some of hydrogen atoms are replaced by fluorine. In an embodiment of the present disclosure, the semifluorinated alkanes include at least one non-fluorinated hydrocarbon segment and at least one perfluorinated hydrocarbon segment, preferably linear or branched alkanes in which a non-fluorinated hydrocarbon segment is linked to a perfluorinated hydrocarbon segment. In detail, the semifluorinated alkanes are (F(CF₂)n(CH₂)mH) type semifluorinated alkanes, or (F(CF₂)n(CH₂)m(CF₂)oF) type semifluorinated alkanes with two perfluorinated segments sandwiching a non-fluorinated segment.

According to some embodiments of the present disclosure, the general formula of the semifluorinated alkanes is RFRH or RFRHRF, where RF represents a perfluorinated segment and RH represents a non-fluorinated segment. The general formula may also be denoted as FnHm and FnHmFo, where F represents a perfluorinated segment, H represents a non-fluorinated segment, and n, m, and o are the number of carbons of each segment. For example, F3H3 represents perfluoropropyl propane. Unless otherwise specified, F3H3 refers to 1-perfluoropropyl propane. Preferably, the semifluorinated alkanes conform to the above-mentioned general formula, and each segment (n, m, o) is independently 3 to 20 carbon atoms.

In some preferred embodiments of the present disclosure, the semifluorinated alkanes are of an RFRH structure, and RF and RH each are of a linear structure, and each includes 3 to 20 carbon atoms. Preferably, RF includes 4 to 12 carbon atoms, and RH includes 4 to 8 carbon atoms. According to some preferred embodiments of the present disclosure, the semifluorinated alkanes include F4H4, F4H5, F4H6, F4H7, F4H8, F6H2, F6H4, F6H6, F6H7, F6H8, F6H9, and F6H10. Preferably, the semifluorinated alkanes are F6H8.

Without being limited by a specific theory, the inventors believe that the chemical and physiological properties of the semifluorinated alkanes substance are applicable to the ophthalmic pharmaceutical composition of the present disclosure. For example, the substance is stable and colorless, and has a density of about 1.1-1.7 g/cm³ and a surface tension as low as 19 mN/m. The RFRH semifluorinated alkanes are insoluble in water, but have certain amphiphilicity, and the longer the non-fluorinated segment, the more lipophilic the RFRH semifluorinated alkanes become. In addition, The semifluorinated alkanes have a refractive index that is close to that of tears (about 1.333), and thus have little influence on vision and will not cause blurred vision.

Specifically, the oil phase may be selected from the group consisting of triglyceride oil, mineral oil, medium-chain triglycerides (MCTs), oily fatty acid, isopropyl myristate, oily fatty alcohol, sorbitan ester, oily sucrose ester, or any other oily substances that are physiologically acceptable to the eyes, preferably the medium-chain triglycerides (MCTs). Without being limited by a specific theory, MCTs help to maintain the lipid layer of a tear film, and can enhance the stability of the lipid layer when added to the ophthalmic pharmaceutical composition. In addition, MCTs can be digested and absorbed more quickly in human bodies. Moreover, MCTs is liquid at room temperature, and their nonpolarity and hydrophobicity can help oily substances to dissolve under a drug delivery system. In addition, with the low viscosity, MCTs are allowed to extend easily on a surface, thereby avoiding expulsion by blink reflex. In addition, the MCTs may be also formed into a stable emulsion, which is beneficial for pharmaceutical applications, particularly in ophthalmic drugs.

Specifically, in the excipient, the concentration of the oil phase is less than or equal to about 50% w/v, for example, 10% w/v, 15% w/v, 20% w/v, 25% w/v, 30% w/v, 35% w/v, 40% w/v, 45% w/v, or 50% w/v.

Without being limited by a specific theory, the present disclosure is particularly suitable for water-insoluble drugs/active ingredients, and these ingredients are quite challenging for the preparation of ophthalmic formulas. As described herein, if the solubility of a compound falls under the categories of "slightly soluble", "sparingly soluble", "very sparingly soluble" or "practically insoluble" (according to the European Pharmacopoeia, 6^{th} Edition), then the compound is considered water-insoluble. Specifically, for example, the solubility of active ingredients in water at neutral pH 6.0 to 8.0 and room temperature of 15 to 25°C is less than about 1 mg/mL.

According to some embodiments of the present disclosure, in order to improve the solubility of the active ingredients, an additional liquid excipient may also be added to ensure that the active ingredients are completely dissolved. Specifically, the additional liquid excipient is a cosolvent and/or a solvent. The cosolvent is preferably an organic cosolvent, for example, oily substances such as glyceride oil, liquid wax, or liquid paraffin, or other organic solvents highly biocompatible. According to some embodiments of the present disclosure, the cosolvent may be selected from organic solvents such as glycerol, propylene glycol, polyethylene glycol, and ethanol.

In a specific embodiment, F6H8 and MCTs are used as the semifluorinated alkanes and the oil phase, respectively. Table 1 provides a comparison of physical properties between F6H8, MCTs, and water. Among these physical properties, surface tension, viscosity and refractive index are key parameters that affect the performance of eye drops in eyes.

**Table 1**

| Physical properties | F6H8 | MCTs | Water |
|---|---|---|---|
| Density (g/cm³) | 1.3 | 0.93-0.96 | 1 |
| Surface tension (mN/m) | 19.63 | 31.26±0.18 | 72 |
| Viscosity (mPa·s) | 3.44 (25°C) | 27-33 (20°C) | 1 (20°C) |
| Melting point (°C) | -5 | -5 | 0 |
| Boiling point (°C) | 223 | 300 | 100 |
| Refractive index (20°C) | 1.333 | 1.440-1.452 | 1.33 |

F6H8 and MCTs used in the present disclosure are both close to water in refractive index. Therefore, it can be inferred that the composition of the present disclosure can better avoid significant differences between ingredients and human tears, thereby avoiding blurred vision.

### [Example 1]

In order to evaluate the compatibility between MCTs and F6H8, the oil phase was used as a solute, and semifluorinated alkanes (SFAs) were used as a liquid vehicle serving as a solvent to prepare excipients having different concentrations of the oil phase. As shown in Table 1, the excipients at all oil phase concentrations are clear and transparent.

**Table 2**

| Concentration of the Oil Phase in the Excipient | 10% w/v | 20% w/v | 30% w/v | 50% w/v |
|---|---|---|---|---|
| Appearance | Clear and transparent | Clear and transparent | Clear and transparent | Clear and transparent |

### [Example 2]

In Example 2 of the present disclosure, 5 mg/mL LBN was selected as an active ingredient for an initial experiment. 5 mg/mL LBN was added to neat MCTs, heated to 80°C and shaken evenly to ensure complete dissolution, and was then cooled at room temperature. The saturated solubility of LBN in neat MCTs was about 3.4 mg/mL as determined by HPLC.

In order to confirm the stability of LBN in an excipient formed from MCTs dissolved in F6H8, and alleviate the eye irritation that may be caused by MCTs in a final product, the present disclosure reduces the concentration of MCTs. The target solubility was set to be twice the concentration of LBN in commercially available product VYZULTA. The product contained 0.24 mg/mL LBN. Therefore, the target was set to be higher than 0.48 mg/mL.

Specifically, in the excipient, the concentration of the oil phase was 10% w/v, 15% w/v, 20% w/v, 25% w/v, 30% w/v, 35% w/v, 40% w/v, 45% w/v, and 50% w/v; preferably 10% w/v, 15% w/v, and 30% w/v, and the solubility of LBN was 0.19 mg/mL, 0.56 mg/mL, and 1.21 mg/mL, respectively (Table 3). In a further embodiment, the concentration employed in the present disclosure was 15% w/v, and the corresponding solubility of LBN was 0.56 mg/mL.

**Table 3**

| Concentration of the Oil Phase in the Excipient | 10% | 15% | 30% |
|---|---|---|---|
| Appearance | Turbid | Clear and transparent | Clear and transparent |
| LBN solubility (mg/mL) | 0.19 | 0.56 | 1.21 |

### [Example 3]

After confirming that the saturated solubility of LBN in neat MCTs was 3.4 mg/mL, ophthalmic pharmaceutical compositions of 1 mg/mL LBN dissolved in neat MCTs, 1 mg/mL LBN dissolved in an excipient having an MCTs concentration of 50% w/v, and 1 mg/mL LBN dissolved in an excipient having an MCTs concentration of 30% w/v were prepared for initial stability testing at different excipient ratios, to confirm whether reducing the concentration of MCTs would affect stability, and were stored at 25°C and 40°C for long periods (1 week, 2 weeks, 1 month, 3 months, and 6 months). The results are shown in Table 4. Specifically, the effective concentration value (unit: % w/v) represents the stability results, and more specifically, the magnitude of the effective concentration value is positively correlated with stability.

**Table 4**

| Conditions | | T0 | 1 week | 2 weeks | 1 month | 3 months |
|---|---|---|---|---|---|---|
| 1 mg/mL LBN dissolved in neat MCTs | 25°C, RH 65% (Long term) | 96.12% | 98.11% | 95.31% | 101.13% | 103.35% |
| | 40°C, RH 75% (Accelerated) | | 96.09% | N/A | 100.02% | 104.22% |
| 1 mg/mL LBN dissolved in an excipient having an MCTs concentration of 50% w/v | 25°C, RH 65% (Long term) | 98.22% | 101.26% | 97.33% | 100.12% | 103.41% |
| | 40°C, RH 75% (Accelerated) | | 100.13% | N/A | 101.03% | 110.21% |
| 1 mg/mL LBN dissolved in an excipient having an MCTs concentration of 30% w/v | 25°C, RH 65% (Long term) | 100.02% | 97.21% | 97.18% | 99.22% | 104.13% |
| | 40°C, RH 75% (Accelerated) | | 98.31% | N/A | 100.12% | 110.31% |

According to the stability data in Table 4, it can be seen that the stability data of formulations of 1 mg/mL LBN in neat MCTs and 1 mg/mL LBN in an excipient having an MCTs concentration of 30% w/v showed little change from T0 to 3 months at 25°C and 40°C. However, some data fluctuated within 10%, which may be attributed to unverified analytical methods, including a sample preparation process.

### [Example 4]

In Example 4 of the present disclosure, based on the data in Table 4, the stability of 0.24, 0.12 and 0.06 mg/mL LBN dissolved in an excipient having an MCTs concentration of 30% w/v was evaluated, and the time and temperature conditions were the same as above.

**Table 5**

| Conditions | | T0 | 1 week | 2 weeks | 1 month | 3 months |
|---|---|---|---|---|---|---|
| 0.24 mg/mL LBN dissolved in an excipient having an MCTs concentration of 15% w/v | 25°C, RH 65% (Long term) | 108.33% | 116.67% | 108.33% | 112.50% | 116.67% |
| | 40°C, RH 75% (Accelerated) | | 112.50% | N/A | 116.67% | 116.67% |
| 0.12 mg/mL LBN dissolved in an excipient having an MCTs concentration of 15% w/v | 25°C, RH 65% (Long term) | 91.67% | 108.33% | 91.67% | 100.00% | 91.67% |
| | 40°C, RH 75% (Accelerated) | | 100.00% | N/A | 100.00% | 83.33% |
| 0.06 mg/mL LBN dissolved in an excipient having an MCTs concentration of 15% w/v | 25°C, RH 65% (Long term) | 100.00% | 100.00% | 83.33% | 100.00% | 66.67% |
| | 40°C, RH 75% (Accelerated) | | 83.33% | N/A | 100.00% | 66.67% |

As shown in Table 5, the fluctuation of 0.06 to 0.24 mg/mL LBN dissolved in an excipient having an MCTs concentration of 15% w/vis about 20% on average, and the lower the concentration, the greater the fluctuation in three months, the worse the stability of LBN.

### [Example 5]

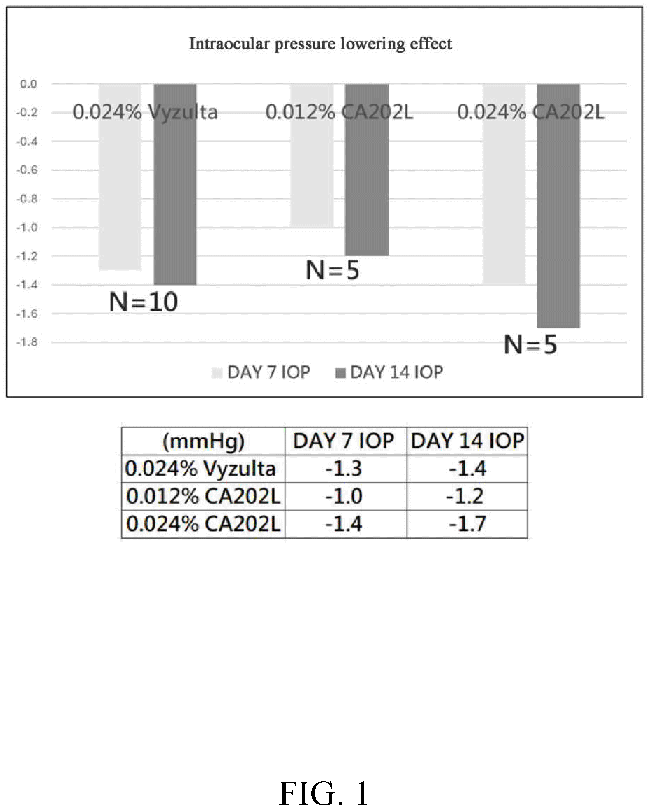

Referring to FIG. 1 to FIG. 4 collectively, in this example, 0.012% w/v and 0.024% w/v LBN in CA202L (experimental number, and ingredient: 0.012% w/v or 0.024% w/v LBN dissolved in an excipient having an MCTs concentration of 15% w/v) and commercially available ophthalmic drug VYZULTA (0.024% w/v LBN) were tested for the effects of lowering intraocular pressure and alleviating dry eye symptoms, side effects and the red eye severity. Subjects were administered with one drop in each ear once nightly, followed by intraocular pressure (IOP) at Day 7 and Day 14, and a self-rating dry eye symptom scale was completed and the red eye severity was assessed. Specifically, the self-rating dry eye symptom scale refers to an ocular surface disease index (OSDI) scale, which is a self-completion questionnaire tool used for assessing the impact of ocular surface diseases (especially the dry eye symptoms) on daily living of patients. The scale primarily focuses on the following three aspects: visual function, for example, difficulties with reading, night driving, computer use, or watching TV; eye symptoms, for example, the frequency of discomfort symptoms such as eye dryness, stinging, foreign body sensation, and fluctuations in vision; and environmental triggers, including the degree of discomfort of the eyes in windy, air-conditioning, or dry environments. The OSDI scale is administered by asking the subjects to rate each question on a scale from 0 to 4 based on the frequency of symptoms or difficulties: 0 = Never, 1 = Occasionally, 2 = Sometimes, 3 = Frequently, 4 = Always. Afterwards, the sum of all scores was multiplied by 25, and the resulting product was divided by the number of valid responses to obtain a final score. The final score of 0 to 12 indicated no dry eye symptom, the final score of 13 to 22 indicated mild dry eye symptom, the final score of 23 to 32 indicated moderate dry eye symptom, and the final score of 33 to 100 indicated severe dry eye symptom.

There were a total of 10 subjects. The experimental conditions were as follows: left eyes of all 10 subjects were instilled with 0.024% w/v LBN in VYZULTA, as a control group; right eyes of 5 of the subjects were instilled with 0.012% w/v LBN in CA202L; and right eyes of the other 5 subjects were instilled with 0.024% w/v LBN in CA202L. The results are shown in FIGS. 1, 2 and 3.

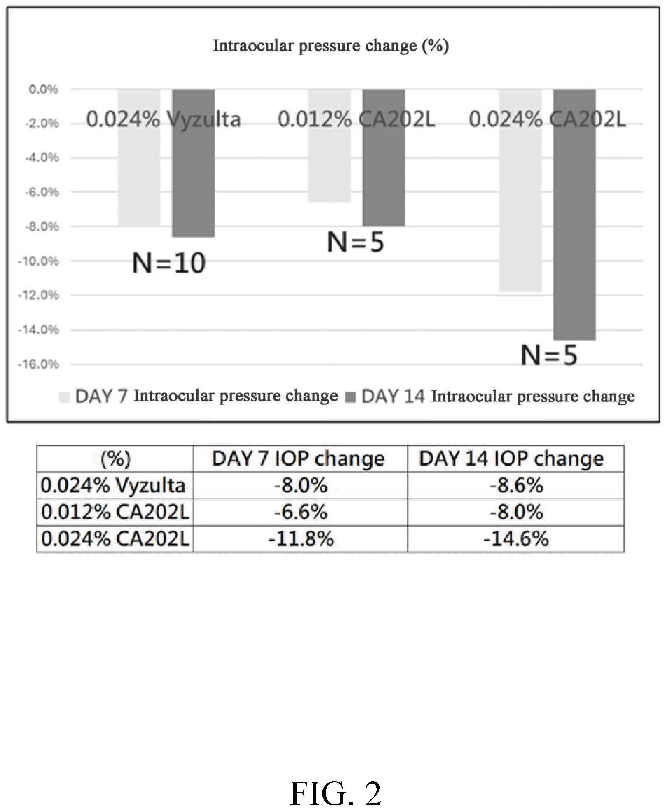

It can be seen from the results that after 14 days of use, 0.012% w/v CA202L and 0.024% w/v VYZULTA have comparable intraocular pressure lowering effects, while 0.024% w/v CA202L is significantly better than 0.024% w/v VYZULTA. Therefore, it is obvious that the ophthalmic pharmaceutical composition of the present disclosure can produce the efficacy similar to those of commercially available drugs with a small content of active ingredients, and when both of them have the same active ingredient content, the composition of the present disclosure has a more excellent effect on increasing the intraocular pressure (FIGS. 1 and 2).

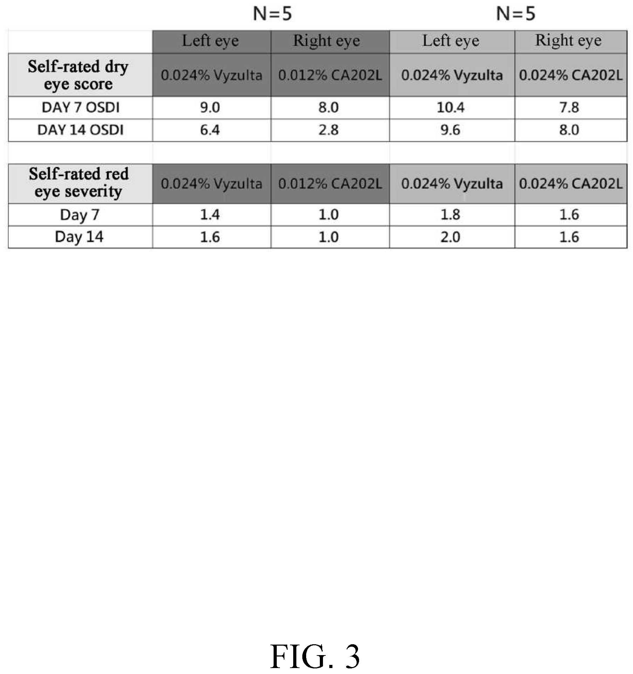

In addition, in the assessment of side effects of dry eye symptoms and eye redness, according to self-rating of the subjects, when both CA202L and VYZULTA are administered, the dry eye score and red eye severity of CA202L are lower than those of VYZULTA, which indicates that the formula of CA202L of the present disclosure brings smaller side effects than VYZULTA (FIG. 3).

In another aspect, the present disclosure provides use of the ophthalmic pharmaceutical composition, specifically use of the ophthalmic pharmaceutical composition for prevention, treatment, or alleviation of eye diseases in an individual, where the eye diseases include elevated intraocular pressure-related symptoms, glaucoma, dry eye symptoms, eye redness or non-arterial anterior ischemic optic neuropathy (NAION).

As mentioned above, for each of the causes of these eye diseases, the effects of prevention and treatment is achieved by lowering intraocular pressure. The dry eye symptoms include: eye dryness, photophobia, eye redness with bloodshot appearance, eye soreness, ocular foreign body sensation, stringy eye discharge, blurred vision, and/or poor vision.

The method for treating or reducing retinal ganglion cell (RGC) damage caused by non-arterial anterior ischemic optic neuropathy (NAION) is to administer the ophthalmic pharmaceutical composition to an individual by contact with the eyes or injection into the eyes.

The prevention, treatment, or alleviation of non-arterial anterior ischemic optic neuropathy (NAION) includes: administering the ophthalmic pharmaceutical composition to an individual by contact with the eyes or injection into the eyes, where the individual is an animal body, for example, rates, mice, or humans.

When the ophthalmic pharmaceutical composition was administered to the individual by contact with the eyes, the concentration of the ophthalmic pharmaceutical composition was 0.006% w/v to 0.040% w/v, and the ophthalmic pharmaceutical composition was administered three times daily for three weeks. Specifically, the concentration was 0.006% w/v, 0.010% w/v, 0.015% w/v, 0.020% w/v, 0.025% w/v, 0.030% w/v, 0.035% w/v or 0.040% w/v.

When the ophthalmic pharmaceutical composition was administered to the individual by injection into the eyes, the concentration of the ophthalmic pharmaceutical composition was 0.04% w/v, and a single injection was employed.

### [Example 6]

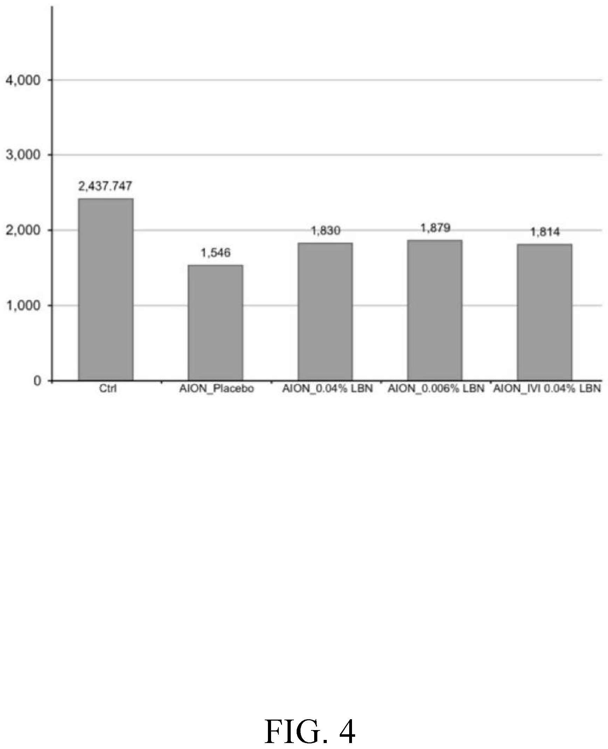

In this example of the present disclosure, the effects of 0.006% w/v and 0.040% w/v LBN in CA202L (experimental number, ingredient: 0.006% w/v or 0.040% w/v LBN dissolved in an excipient having an MCTs concentration of 15% w/v) were determined using a mouse model (NAION C57BL/6). The results are shown in FIG. 4.

Regarding the establishment of the NAION C57BL/6 mouse model, a photochemical thrombosois method was used. In this method, 1.25 mL of Rose Bengal was injected into the mouse tail vein, and then NY-YAG green laser (300 µm spot diameter, 50 mW power, 1 second of duration, and 15 spots) was used for NAION induction.

The experimental flows were as follows: after being induced, the mice were administrated with placebo, 0.006% w/v CA202L eye drops three times a day for three weeks, 0.040% w/v CA202L eye drops three times a day for three weeks, and a single intraocular injection of 0.040% w/v CA202L. Then Brn3A (biomarker)+ retinal ganglion cells (RGC) were counted after immunostaining (unit: cells/mm²).

As shown in FIG. 4, the vertical axis represents the Brn3A+RGC count results, and the horizontal axis represents a control group, a placebo group, a 0.040% w/v eye drops group, a 0.006% w/v eye drops group, and a 0.04% w/v intraocular injection group. It can be seen from the results that after the mice in the placebo group were induced, the count decreased from 2437 in control group to 1546, and the number of RGC cells decreased by about 36%. If 0.006% w/v or 0.040% w/v of CA202L eye drops were used, or eyeballs were administrated with a single injection of 0.04% w/v CA202L after induction, 27% to 36% RGC protection effect can be achieved, demonstrating that CA202L has a significant effect in reducing the RGC damage caused by NAION (the number of cells was only reduced by about 23% to 26%, and the RGC count was 1814 to 1830).

[Biomaterial Deposit]

## Claims

1. An ophthalmic pharmaceutical composition, comprising:
0.006% w/v to 0.121% w/v of latanoprostene bunod (LBN) or a pharmaceutically acceptable salt thereof;
at least one excipient, comprising an oil phase and a liquid vehicle of semifluorinated alkanes (SFAs),
wherein the composition is microbiologically stable, and is water-free and preservative-free;
wherein the composition is formulated into a solution, an emulsion, or a suspension;
wherein in the excipient, the concentration of the oil phase is 10% w/v to 50% w/v; and
wherein the oil phase is medium-chain triglyceride (MCT).

2. The ophthalmic pharmaceutical composition according to claim 1, comprising 0.006% w/v to 0.1% w/v of latanoprostene bunod (LBN) or a pharmaceutically acceptable salt thereof.

3. The ophthalmic pharmaceutical composition according to claim 1, wherein in the excipient, the concentration of the oil phase is 15% w/v, and the solubility of the latanoprostene bunod in the excipient is 0.56 mg/mL.

4. The ophthalmic pharmaceutical composition according to claim 1, wherein in the excipient, the concentration of the oil phase is 15% w/v to 30% w/v.

5. The ophthalmic pharmaceutical composition according to claim 1, wherein the refractive index of the semifluorinated alkanes ranges from 1.29 to 1.35 at 20°C.

6. The ophthalmic pharmaceutical composition according to claim 1, wherein the semifluorinated alkanes are linear or branched alkanes in which a non-fluorinated hydrocarbon segment is linked to a perfluorinated hydrocarbon segment.

7. The ophthalmic pharmaceutical composition according to claim 6, wherein the general formula of the semifluorinated alkanes is RFRH, wherein RF represents the perfluorinated hydrocarbon segment, RH represents the non-fluorinated hydrocarbon segment, and RF and RH are each of a linear structure, and each have 3 to 20 carbon atoms.

8. The ophthalmic pharmaceutical composition according to claim 7, wherein the perfluorinated hydrocarbon segment comprises 4 to 12 carbon atoms, and the non-fluorinated hydrocarbon segment comprises 4 to 10 carbon atoms.

9. The ophthalmic pharmaceutical composition according to claim 1, wherein the semifluorinated alkanes are selected from the group consisting of F4H4, F4H5, F4H6, F4H7, F4H8, F6H2, F6H4, F6H6, F6H7, F6H8 (perfluorohexyloctane), F6H9, and F6H10.

10. The ophthalmic pharmaceutical composition according to claim 1, wherein the excipient further comprises: a cosolvent and/or a solvent.

11. The ophthalmic pharmaceutical composition according to claim 9, wherein the cosolvent is an organic solvent selected from glycerol, propylene glycol, polyethylene glycol, and ethanol.

12. An ophthalmic pharmaceutical composition according to any one of claims 1 to 11 for use in the treatment, prevention, or alleviation of eye diseases in an individual, wherein the eye diseases comprise elevated intraocular pressure-related symptoms, glaucoma, or non-arterial anterior ischemic optic neuropathy (NAION).

13. The ophthalmic pharmaceutical composition for use according to claim 12,
wherein the treatment, prevention, or alleviation of glaucoma comprises lowering intraocular pressure in the individual.

14. The ophthalmic pharmaceutical composition for use according to claim 12,
wherein the elevated intraocular pressure-related symptoms comprise dry eye symptoms or eye redness as a side effect.

15. The ophthalmic pharmaceutical composition for use according to claim 14, wherein the dry eye symptoms comprise eye dryness, photophobia, eye redness with bloodshot appearance, eye soreness, ocular foreign body sensation, stringy eye discharge, blurred vision, and/or poor vision.

16. The ophthalmic pharmaceutical composition for use according to claim 12, wherein the treatment, prevention, or alleviation of non-arterial anterior ischemic optic neuropathy (NAION) comprises:
administering the ophthalmic pharmaceutical composition to an individual by contact with the eyes or injection into the eyes,
wherein when the ophthalmic pharmaceutical composition is administered to the individual by contact with the eyes, the concentration of latanoprostene bunod is 0.006% w/v to 0.040% w/v, and the ophthalmic pharmaceutical composition is administered three times daily for three weeks; and
wherein when the ophthalmic pharmaceutical composition is administered to the individual by injection into the eyes, the concentration of latanoprostene bunod is 0.04% w/v, and a single injection is employed.
